# EUROPEAN PATENT APPLICATION

(11) **EP 4 186 382 A1**
(43) Date of publication of application: **31.05.2023**
(21) Application number: 21845942.8
(22) Date of filing: 22.07.2021
(51) Int. Cl.: A41D 31/30, A61L 26/00, A61K 31/724, A61K 47/69, A61K 9/70, A61K 31/155

(54) **MESH MATERIALS FOR CONTROLLED RELEASE OF ANTIVIRALS, METHOD AND USE**

(30) Priority: 24.07.2020 BR 102020015177
(71) Applicant: Universidade Federal de Minas Gerais, 31270-901 Belo Horizonte (BR); 2Heal Industria e Comercio de Produtos Quimicos Para uso Industrial Ltda, 13211-698 Jundiaí (BR)
(72) Inventor: SINISTERRA MILLÁN, Rubén Dario, 31270-901 Belo Horizonte (BR); CORTÉS SEGURA, Maria Esperanza, 31270-901 Belo Horizonte (BR); MARTINEZ ANDRADE, Alfonso, 31270-901 Belo Horizonte (BR); ANDRÉS GRAJALES RUIZ, Daniel, 31270-901 Belo Horizonte (BR); MOISES IWAMIZU SILVA, Renata, 13211-685 Jundiaí (BR)
(74) Representative: Clarke Modet & Co.
(86) International application number: PCT/IB2021/056655
(87) International publication number: WO 2022/018689

(57) **Abstract**

The present technology refers to mesh materials for the controlled release of antimicrobial and antiviral compounds, with high antiviral activity and efficiency, mainly against the coronavirus. It also refers to the process for producing mesh materials with bis-biguanide:cyclodextrin inclusion compounds in molar ratios in the range of 1:1 to 1:4. The mesh materials are selected from the group consisting of polymers, fibers, non-woven fabric (NWF), fabrics, paper, cellulose, alone or in combination, or a combination of one or more of these mesh materials with adhesives. Mesh materials impregnated, extruded, saturated, coated or laminated with bis-biguanide:cyclodextrin inclusion compounds have been shown to be effective in inactivating/destroying viral particles. In fabrics, the inclusion compounds showed virucidal activity for all viruses of the coronavirus group for a contact time from 2 minutes, having its effect prolonged for a period greater than 30 days. Mesh materials for controlled release of antivirals showed activity up to 1,000 times more effective compared to the use of non-encapsulated chlorhexidine.

## Description

The present technology refers to mesh materials for the controlled release of antimicrobial and antiviral compounds, with high antiviral activity and efficiency, mainly against the coronavirus. It also refers to the process for producing mesh materials with bis-guanide:cyclodextrin inclusion compounds in molar ratios in the range of 1:1 to 1:4. The mesh materials are selected from the group consisting of polymers, fibers, non-woven fabric (NWF), fabrics, paper, cellulose, alone or in combination, or a combination of one or more of these mesh materials with adhesives. Mesh materials impregnated, extruded, saturated, coated or laminated with bis-guanide:cyclodextrin inclusion compounds have been shown to be effective in inactivating/destroying viral particles. In fabrics, the inclusion compounds showed virucidal activity for all viruses of the coronavirus group for a contact time from 2 minutes, having its effect prolonged for a period greater than 30 days. Mesh materials for controlled release of antivirals showed activity up to 1,000 times more effective compared to the use of non-encapsulated chlorhexidine.

The most common meshes include aluminum foil, metal, paper, textiles, plastic film, and wires. Processes that allow the addition of additives on material bases include: coating, stripping, printing, dyeing, extruding, impregnating, saturating, atomizing, electroplating and laminating. Non-woven fabric (NWF) materials, for example, are used as components in a variety of products, such as, but not limited to, disposable diapers and absorbent pants, sanitary napkins, personal skin cleansing wipes (such as wet wipes), adhesive tapes, personal protective equipment, disposable towels, packaging, cleaning cloths, which are typically formed from artificial or synthetic raw materials, short fibers and heat bonded, not needing to be woven.

Upon evaluating the state of the art, it was possible to verify that there are some technologies using mesh materials for controlled release of active principles, as presented below.

In the area of food packaging, there is a constant search for solutions that can increase their durability by preventing the proliferation of microorganisms (fungi and bacteria). Solutions conquered through the functionalization of the material bases with a compound of inclusion of actives with antimicrobial activity and cyclodextrins are available. Patent JPH04325069 presents a coating for a synthetic resin or NWF film, intended to be used in food packaging, adding the hinokitiol:cyclodextrin inclusion compound in polymers and NWF.

The work carried out by Kayaci and collaborators shows the increase of antimicrobial activity in nanofiber of polylactic acid (PLA), used in the production of plastic films for packaging food, when inclusion compounds of triclosan:cyclodextrins are added in the electrospinning process, in comparison with the addition of the triclosan active in isolated form (Fatma Kayaci, Ozgun C.O. Umu, Turgay Tekinay and Tamer Uyar, J. Agric. Food Chem. 2013, 61, 3901-3908).

The technology described in patent document US 6,267,975 refers to a disposable product for personal cleansing, substantially dry, comprising: a) a water-insoluble substrate comprising: 1) a first layer that exhibits a higher softness ratio to about 1.1; 2) a second layer disposed adjacent to said first layer, said second layer exhibiting a softness ratio of less than about 1.2; b) a cleaning component disposed adjacent to said first and second layers, said component comprising from about 10% to about 1000%, by weight, of the water-insoluble substrate, of a surfactant. It further relates to a disposable article substantially dry and suitable for conditioning, in which the above-described article comprises a component of therapeutic benefit or useful for personal cleansing applications, in particular for the skin and hair.

The patent US 6,428,799 describes a disposable and substantially dry personal care article, suitable for cleaning and/or therapeutic treatment, comprising a water-insoluble substrate comprising a non-abrasive, high and low-density batting layer, which comprises synthetic fibers and wherein said batting layer exhibits a number of physical properties, individually or in combination.

The technology described in the patent document AU2016223346 describes textiles with antimicrobial properties. It refers to a method of manufacturing a textile material with antimicrobial compounds in such a way as to chemically bond or fix inclusion compounds to the textile material and the treated textile material, which acts as a disinfectant or sterilizer alone. The treated textile material exhibits durability and non-leaching properties. It further refers to a device for purifying water, which can operate based on gravity and without electricity.

Patent US2002/0034616 describes a mesh material comprising multiple micropores. It is an objective of the technology to provide a mesh material for combinatorial chemistry experimentation that is substantially thin and flexible and with distinct regions that can accommodate fluids and/or solids.

The mesh materials have a wide range of potential uses in durable and disposable articles, but are particularly suitable for use in disposable absorbent articles such as disposable diapers, incontinence briefs, training pants, sanitary napkins and the like.

The US patent US 5,668,097 refers to a stable odor absorbent aqueous composition for use on inanimate surfaces. The composition comprises from about 0.1% to about 5%, by weight of the composition, of solubilized, water-soluble, uncomplexed cyclodextrin, an effective amount of a solubilized, water-soluble, water-soluble antimicrobial preservative greater than about 0.3%, optional perfume, and an aqueous vehicle.

The mesh materials can be used in air conditioning filters. Among pollutants, the airborne particles, including fine particles and bioaerosols, have gained increasing attention as they can be easily transported from one place to another. This is because the size of bioaerosols varies from submicron in size (< 0.01 µm) to greater than 100 µm (Smith, G. J. D., Vijaykrishna, D., Bahl, J., Lycett, S. J., Worobey, M., Pybus, O. G., Ma, S. K., Cheung, C. L., Raghwani, J., Bhatt, S., Peiris, J. S. M., Guan, Y., Rambaut, A., Nature, 459, 1122 (2009)). By considering the size of the particles, they can be suspended in the atmosphere and kinetically stable for a long time.

Several technologies have been developed for air purification, such as electrostatic precipitation, cold plasma, wet washing, cyclonic air filtration (venturi) and physical filters (glass fibers, melt-blown fibers, spun-bonded fibers and ceramic filters), (Brincat, J. P.; Sardella, D.; Muscat, A.; Decelis, S.; Grima, J. N., Valdramidis, V., Gatt, R., Trends Food Sci. Technol., 50, 175 (2016). 6. A; Joubert, J. -C. Laborde, L. Bouilloux, S. Chazelet, D. Thomas, Chem. Eng. J., 166, 616 (2011)). Filtration is the most interesting technology for removing airborne particles, as it offers simple operation, low energy consumption and low operating and investment cost. The capabilities of physical air filtration lead to an increase in publications in this field of application.

Some anteriorities were found in the state of the art referring to non-woven fabric materials or polymers or nanofibers carriers of antimicrobial and antiviral compounds. The US patent US 9,549,949 describes an antiviral agent that contains as active ingredient a particle of at least one type of iodide formed by ionic iodine and a cation of a chemical element from group 8 to 15, between periods 4 and 6, of table periodic or at least formed by a type of compound containing monovalent copper (Cu(I)). The antiviral agent can be incorporated into a variety of products and has the ability to inactivate a wide range of viruses.

The technology in US patent US 8,172,925 claims a cabin air filter with maximum functionality that includes a fine dust collection filter layer, an oxidation catalyst filter layer to convert nitrogen monoxide to nitrogen dioxide, and an adsorption filter layer for adsorbing nitrogen dioxide and volatile organic compounds, wherein antimicrobial nanoparticles are applied to at least one dust collection filter layer, oxidation catalyst filter layer, and adsorption filter layer. This cabin air filter has dust collection, denitrification, deodorization and antimicrobial functions, and can be used in various ways for air purification in a limited space such as a vehicle.

Reported works in the state of the art show that nanomaterials can be applied as a coating in, for example, starch-based matrices for the production of papers with antimicrobial property. A work carried out by Martins and collaborators shows that zinc oxide nanoparticles in nanofibrillated cellulose compounds, in a concentration lower than 0.03%, presented bacteriostatic and/or bactericidal activity against Gram-positive and Gram-negative bacteria (Martins, N. C. T.; Freire, C. S. R., Neto, C. P., Silvestre, A. J. D., Causio J., Baldi, G., Sadocco, P., Trindade, T. Colloids and Surfaces A: Physicochem. Eng. Aspects 417 (2013) 111-119).

Non-limiting examples of materials that can be spun into filaments are commonly seen in the state of the art. Filaments are normally considered to be continuous or substantially continuous in nature. They are relatively longer than fibers and can be single or multicomponent. Examples of filaments include melt blown and/or spun filaments such as starch, cellulose, hemicellulose and their respective derivatives, chitin, chitosan, polyisoprene, peptides, polyhydroxyalkanoates and synthetic polymers including but not limited to thermoplastic polymer filaments and polyolefin copolymers. Useful papermaking fibers for the purposes described herein include cellulosic fibers commonly known as wood pulp fibers.

Among the most robust antimicrobial halogenated compounds that can function as antivirals are 1,1'-hexamethylene-bis(5-(p-chlorophenyl)-biguanide), commonly known as chlorhexidine, and its salts, and poly-(hexamethylene-biguanide) hydrochloride, used as an active principle present in the commercialized products Cosmoci^{®} CQ^{®} and Vantocil^{®} IB.

The present technology, for which protection is claimed, contemplates that cleaning and viral disinfection can occur by mixing one or more materials in a mesh as matrices for the controlled release of inclusion compounds with bis-guanide and cyclodextrin. There can be used commercially available laminated products that can be adhered to NWF, the mesh materials and their combinations described in the present technology to serve as devices for the controlled release of antiviral compounds, such as the chlorhexidine:cyclodextrin inclusion compound in different molar ratios.

Laminated materials can be joined in any suitable way, such as, among others, ultrasonic bonding, adhesive, glue, melt bonding, heat bonding, thermal bonding and combinations thereof. An alternative may be a laminate including one or more layers of non-woven mesh materials and one or more layers of film. Examples of such optional films include, but are not limited to, polyolefin films such as polyethylene and non-woven fabrics film or mixtures thereof.

Although the biguanides are described in the state of the art (US2005/0176611A1) as antimicrobial compounds, in the present technology the bis-biguanides complexed with cyclodextrin have a long-lasting antiviral effect, and can be incorporated into mesh materials.

Cyclodextrins are cyclic oligosaccharides that include six, seven or eight glucopyranose units. Due to steric interactions, cyclodextrins (CDs) form a structure in the form of a truncated cone with a non-polar internal cavity. These are chemically stable compounds that can be modified in a regioselective manner. Cyclodextrins act as hosts to form complexes with multiple guests in their cavity.

CDs were used in the state of the art for the solubilization and encapsulation of drugs, perfumes and fragrances, as described by Szeitli (Szeitli, J., Chemical Reviews, (1998), 98, 1743-1753. Szeitli, J., J. Mater. Chem., (1997)). According to detailed studies of toxicity, mutagenicity, teratogenicity and carcinogenicity on cyclodextrins, described by Rajewski & Stella, they showed low toxicity, especially hydroxypropyl-β-cyclodextrin (Rajewski, R. A., Stella, V., J. Pharmaceutical Sciences, (1996), 85, 1142-1169). Except for some high concentrations, which cause damage to erythrocytes, these products are generally not harmful to health.

The use of cyclodextrins as food additives has already been authorized in countries such as Japan and Hungary, and for more specific applications, in France and Denmark. Furthermore, they are obtained from a renewable source of starch degradation. All these characteristics are a great motivation for research results for new applications.

The practical uses of cyclodextrin are classified as follows: 1. Transporters (solubilizers, stabilizers) for biologically-active actives; 2. Enzyme models; 3. Separating agents (for chromatography or batch processes); and, 4. Catalysts and additives and detergents, viscosity modifiers (L. Szente and J. Szetjli, Adv. Drug Deliv. ver. 36 (1999), 17).

CDs are sparingly soluble in water, methanol and ethanol and easily soluble in polar solvents such as dimethylsulfoxide, dimethylformamide, N,N-dimethylacetamide and pyridine. There are numerous research papers in the literature on the effects of increasing the water solubility of poorly water-soluble guests using cyclodextrins via the inclusion compounds in use (Szeitli, J., Chemical Reviews, (1998), 98, 1743-1753. Szetjli, J., J. Mater. Chem., (1997), 7575-587), in addition to the use of cyclodextrins for controlled release of encapsulated compounds in their cavity.

The present technology describes mesh materials for controlled release of high activity and antiviral efficiency, mainly against the coronavirus and the process for impregnation with inclusion compounds of bis-biguanide:cyclodextrins in the range of 1:1 to 1:4, having bis-biguanide and cyclodextrin as antiviral active principles.

The matrices claimed in the present technology are based on mesh materials, such as polymers, fibers, non-woven fabric, fabrics, paper, cellulose, or a combination of these materials with adhesives, comprising modified or controlled release systems of antiviral compounds. The mesh materials present long, thin, flexible films or wires that are capable of absorbing bis-biguanide:cyclodextin inclusion compounds.

The technology shows the surprising effect of the antiviral activity of the chlorhexidine:cyclodextrin complex (chemical compound applied to a textile substrate) and demonstrates the durability of the obtained effect. The mesh material was able to inactivate the coronavirus with a 2-minute microbial death kinetics assessment test (time to kill) and a long-lasting test ("long lasting") of up to ten days, when the textile substrates were submerged in a 1.25% (m/v) solution of the chlorhexidine:beta-cyclodextrin inclusion compound.

Cytotoxicity tests of the mesh materials with chlorhexidine:cyclodextrin inclusion compound in fabrics demonstrated that the materials were not toxic to the tested cell line; therefore, they did not cause damage to living cells at a concentration of at least 1.25% (m/v) of the product. By inhibiting viral infection, the present technology shows that chlorhexidine:cyclodextrin inclusion compounds are effective for the inactivation/destruction of viral particles in fabrics, especially coronaviruses, and, therefore, the present technology claims its use as a potential virucidal agent fabrics and other mesh materials.

The surprising results obtained by the present technology are the fundamental features of the present technology when compared with the state of the art that shows that chlorhexidine does not have or has a little significant antiviral effect.

In the state of the art, no technology was found that combines the use of polymeric mesh materials, fibers, non-woven fabric, fabrics, paper and/or cellulose, or a combination of these materials with adhesives, impregnated, extruded, coated or laminated with inclusion compounds formed by bis-biguanide:cyclodextrin as modified or controlled release systems with antiviral activity.

The technology for which protection is claimed refers to mesh materials with bis-biguanide:cyclodextrin inclusion compounds in molar ratios in the range of 1:1 to 1:4, their preparation process, their use as systems of controlled release of compounds with antiviral activity, mainly against Covid-19. Another feature of the present technology is to include at least one layer of adhesive materials, removable or not, by the surface coating process (coating process) or any other method already known in the state of the art, on one side of the matrices of non-woven fabrics, textiles, polymers, non-degradable or biodegradable polymer films, or papers, making them bases for adhesive materials. The adhesion process between the chosen adhesive and the base material takes place through the coating process, creating a layer of superficial adhesive, with temperatures reaching up to 250 °C. On the opposite side to the applied adhesive, the liquid with the inclusion compounds of chlorhexidine:cyclodextrins as antivirals will be inserted, and this liquid may contain other components, such as varnishes to improve the material mechanical strength or protection against solar radiation, which is a feature of the present technology.

### BRIEF DESCRIPTION OF THE FIGURES

Figure 1 shows the spectrum in the visible ultraviolet region of the release of chlorhexidine in aqueous medium.
Figure 2 shows the fabrics after washing with non-ionic detergents.
Figure 3 shows the linear regression of the transition 505.3 - 353.1.
Figure 4 shows the linear regression of the transition 505.3 - 336.4.
Figure 5 shows the linear regression of the transition 505 - 170.
Figure 6 shows the controlled release curve of chlorhexidine from 2900 fabric sample.
Figure 7 shows the controlled release curve of chlorhexidine from 2724 fabric sample.
Figure 8 shows the study of antimicrobial activity against the bacterium *Escherichia coli* by analysis of the size of the inhibition halo of PL21E06 and PL21E07 materials.

### DETAILED DESCRIPTION OF THE TECHNOLOGY

This technology refers to mesh materials for the controlled release of antimicrobial and antiviral compounds, with high activity and antiviral efficiency, mainly against the coronavirus. It also refers to the process for producing mesh materials with bis-biguanide:cyclodextrin inclusion compounds in molar ratios in the range of 1:1 to 1:4. The mesh materials are selected from the group consisting of polymers, non-woven fabrics (NWF), fabrics, fibers, paper, cellulose, alone or in combination, or a combination of at least one of these mesh materials with adhesives. Mesh materials impregnated, extruded, saturated, coated or laminated with bis-biguanide:cyclodextrin inclusion compounds have been shown to be effective in inactivating/destroying viral particles.

The mesh materials for controlled release of antivirals comprise one or more mesh materials selected from the group consisting of polymers, fibers, non-woven fabric (NWF), fabrics, paper, cellulose, or a combination of one or more of these mesh materials with adhesives, impregnated, extruded, saturated, coated or laminated with bis-biguanide:cyclodextrin inclusion compounds in the range of molar ratios between 1:1 and 1:4.

Bis-biguanide may be selected from the group consisting of chlorhexidine, 1,6-bis-(2-ethylhexylbiguanide-hexane) dihydrochloride, 1,6-di-(*N*1,*N*1'-phenyldiguanide-*N*5,*N*5')-hexane tetrahydrochloride, 1,6-di-(*N*1,*N*1'-phenyl-*N*1,*N*1'-methyldiguanide-*N*5,*N*5')-hexane dihydrochloride, 1,6-di-(*N*1,*N*1'-*o*-chlorophenyldiguanide-*N*5,*N*5')-hexane dihydrochloride, 1,6-di-(*N*1,*N*1'-2,6-dichlorophenyldiguanide-*N*5,*N*5')-hexane dihydrochloride, 1,6-di-[*N*1,*N*1'-beta-(p-methoxyphenyl)-diguanide-*N*5,*N*5']-hexane dihydrochloride, 1,6-di-(*N*1,*N*1'-alpha-methyl-beta-phenyldiguanide-*N*5,*N*5')-hexane dihydrochloride, 1,6-di-(*N*1,*N*1'-p-nitrophenyldiguanide-*N*5,*N*5')-hexane dihydrochloride, omega-di-(*N*1,*N*1'-phenyldiguanide-*N*5,*N*5')- di-n-propyl ether dihydrochloride, omega'-di-(*N*1,*N*1'-*p*-chlorophenyldiguanide-*N*5,*N*5')-di-n-propyl ether tetrahydrochloride, 1,6-di-(*N*1,*N*1'-2,4-dichlorophenyldiguanide-*N*5,*N*5')-hexane tetra-hydrochloride, 1,6-di-(*N*1,*N*1'-*p*-methylphenyldiguanide-*N*5,*N*5')-hexane dihydrochloride, 1,6-di-(*N*1,*N*1'-2,4,5-trichlorophenyldiguanide-*N*5,*N*5')-hexane tetrahydrochloride, 1,6-di-[*N*1,*N*1'-Alpha-(*p*-chlorophenyl)ethyldiguanide-*N*5,*N*5']-hexane dihydrochloride, omega-Mega-di-(*N*1,*N*1'-*p-*chlorophenyldiguanide-*N*5,*N*5')-m-xylene, 1,12-di-(*N*1,*N*1'-*p-*chlorophenyldiguanide*-*N5,*N*5')-dodecane dihydrochloride, 1,10-di-(*N*1,*N*1'-phenyldiguanide-*N*5,*N*5')-decane tetra-hydrochloride, 1,12-di-(*N*1,*N*1'-phenyldiguanide-*N*5,*N*5')-dodecane tetra-hydrochloride, 1,6-di-(*N*1,*N*1'-o-chlorophenyldiguanide-*N*5,*N*5')-hexane dihydrochloride, 1,6-di-(*N*1,*N*1'-p-chlorophenyldiguanide-*N*5,*N*5')-hexane tetrahydrochloride, ethylene *bis*(1-tolyl biguanide), ethylene *bis*(*p*-tolyl biguanide), ethylene *bis*(3,5-dimethylphenyl biguanide), ethylene *bis*(*p*-tert-amylphenyl biguanide), ethylene *bis*(nonylphenyl biguanide), ethylene *bis*(phenyl biguanide), ethylene *bis*(*N*-butylphenyl biguanide), ethylene *bis*(2,5-diethoxyphenyl biguanide), ethylene *bis*(2,4-dimethylphenyl biguanide), ethylene *bis*(o-diphenyl biguanide), ethylene bis (mixed amyl naphthyl biguanide), *N*-butyl ethylene *bis*(phenylbiguanide), trimethylene *bis*(o-tolyl biguanide), *N*-butyl trimethylene *bis*(phenyl biguanide), and the corresponding pharmaceutically acceptable salts of all of the above, such as the acetates, gluconates, hydrochlorides, hydrobromides, citrates, bisulfites, fluorides, polyhalates, *N*-alkylalkylsarcosinates, phosphites, hypophosphites, perfluorooctanoates, silicates, sorbates, salicylates, maleates, tartrates, fumarates, ethylenediaminetetraacetates, iminodiacetates, cinnamates, thiocyanates, arginates, pyrromellites, tetracarboxybutyrates, benzoates, glutarates, monofluorophosphates, and perfluoropropionates and mixtures thereof.

The cyclodextrin may be selected from the group consisting of alpha-cyclodextrin, beta-cyclodextrin, gamma-cyclodextrin, hydroxy-propyl-beta-cyclodextrin, 2,6-o-methyl-beta-cyclodextrin, sulfobutyl-ether-beta-cyclodextrin, radomyl-beta-cyclodextrin, methyl-beta-cyclodextrin and maltosyl-beta-cyclodextrin.

The inclusion compounds may comprise chlorhexidine:cyclodextrin, in the molar ratio range between 1:1 and 1:4.

The mesh materials must be impregnated with at least 0.02% by mass of chlorhexidine.

The process of obtaining mesh materials for controlled release of antivirals comprises the following steps:
a) Preparing an aqueous solution of inclusion compounds of bis-biguanide:cyclodextrin, in the molar ratio range between 1:1 and 1:4 (bis-biguanide:cyclodextrin), at room temperature or up to 80 °C;
b) Impregnating, extruding, saturating, coating or laminating the selected mesh materials from the group consisting of polymers, non-woven fabric (NWF), fabrics, paper or cellulose, alone or in combination, or a combination of these mesh materials with adhesives, with the aqueous solutions of step "a", in final mass concentrations of at least 0.02% of bis-biguanide, for a minimum of 4 seconds, using the methods of exhaustion, impregnation, saturation or spray, dyeing, serigraphy, printing, laundry or atomization followed or not by squeezing and/or fixing;
c) Drying the mesh material obtained in step "b" at room temperature or at a temperature between 70 °C and 200 °C.

In step "a", the aqueous solution may optionally comprise varnishes, dyes or other actives compatible with the inclusion compounds.

In step "a", the cyclodextrin can be selected from the group consisting of alpha-cyclodextrin, beta-cyclodextrin, gamma-cyclodextrin, hydroxy-propyl-beta-cyclodextrin, 2,6-o-methyl-beta-cyclodextrin, sulfobutyl - ether-beta-cyclodextrin, radomyl-beta-cyclodextrin, methyl-beta-cyclodextrin and maltosyl-beta-cyclodextrin, and the bis-biguanide may be selected from the group consisting of chlorhexidine, 1,6-bis-(2-ethyl-hexylbiguanide-hexane) dihydrochloride, 1,6-di-(*N*1,*N*1'-phenyldiguanide-*N*5,*N*5')-hexane dihydrochloride, 1,6-di-(*N*1,*N*1'-phenyl-*N*1,*N*1'-methyldiguanide-*N*5,*N*5')-hexane dihydrochloride, 1,6-di-(*N*1,*N*1'-*o*-chlorophenyldiguanide-*N*5,*N*5')-hexane dihydrochloride, 1,6-di-(*N*1,*N*1'-2,6-dichlorophenyldiguanide-*N*5,*N*5')-hexane dihydrochloride, 1,6-di-[*N*1,*N*1'-beta-(*p*-methoxyphenyl)-diguanide-*N*5,*N*5']-hexane dihydrochloride, 1,6-di-(*N*1,*N*1'-alpha-methyl-beta-phenyldiguanide-*N*5,*N*5')-hexane dihydrochloride, 1,6-di-(*N*1,*N*1'-*p*-nitrophenyldiguanide-*N*5,*N*5')-hexane dihydrochloride, omega-di-(*N*1,*N*1'-phenyldiguanide-*N*5,*N*5')-di-n-propyl ether dihydrochloride, omega'-di-(*N*1,*N*1'-*p*-chlorophenyldiguanide-*N*5,*N*5')-di-n-propyl ether tetrahydrochloride, 1,6-di-(*N*1,*N*1'-2,4-dichlorophenyldiguanide-*N*5,*N*5')-hexane tetra-hydrochloride, 1,6-di-(*N*1,*N*1'-*p*-methylphenyldiguanide-*N*5,*N*5')-hexane dihydrochloride, 1,6-di-(*N*1,*N*1'-2,4,5-trichlorophenyldiguanide-*N*5,*N*5')-hexane tetrahydrochloride, 1,6-di-[*N*1,*N*1'-Alpha-(*p-*chlorophenyl)ethyldiguanide-*N*5,*N*5']-hexane dihydrochloride, omega-Mega-di-(*N*1,*N*1'-*p*-chlorophenyldiguanide-*N*5,*N*5')-m-xylene, 1,12-(*N*1,*N*1'-*p-*chlorophenyldiguanide-*N*5,*N*5')-dodecane dihydrochloride, 1,10-di-(*N*1,*N*1'-phenyldiguanide-*N*5,*N*5')-decane tetrahydrochloride, 1,12-di-(*N*1,*N*1'-phenyldiguanide-*N*5,*N*5')-dodecane tetra-hydrochloride, 1,6-di-(*N*1,*N*1'-o-chlorophenyldiguanide-*N*5,*N*5')-hexane dihydrochloride, 1,6-di-(*N*1,*N*1'-p-chlorophenyldiguanide-*N*5,*N*5')-hexane tetra-hydrochloride, ethylene *bis*(1-tolyl biguanide), ethylene *bis*(*p*-tolyl biguanide), ethylene *bis*(3,5-dimethylphenyl biguanide), ethylene *bis*(*p*-tert-amylphenyl biguanide), ethylene *bis*(nonylphenyl biguanide), ethylene *bis*(phenyl biguanide), ethylene *bis*(N-butylphenyl biguanide), ethylene *bis*(2,5-diethoxyphenyl biguanide), ethylene *bis*(2,4-dimethylphenyl biguanide), ethylene *bis*(o-diphenylbiguanide), ethylene *bis*(mixed amyl naphthyl biguanide), *N*-butyl ethylene *bis*(phenyl biguanide), trimethylene bis(*o*-tolyl biguanide), *N*-butyl trimethylene *bis*(phenyl biguanide), and the corresponding pharmaceutically acceptable salts of all of the items above, such as acetates, gluconates, hydrochlorides, hydrobromides, citrates, bisulfites, fluorides, polyhalates, *N*-alkylalkylsarcosinates, phosphites, hypophosphites, perfluorooctanoates, silicates, sorbates, salicylates, maleates, tartrates, fumarates, ethylenediaminetetraacetates, iminodiacetates, cinnamates, thiocyanates, arginates, pyrromellites, tetracarboxybutyrates, benzoates, glutarates, monofluorophosphates, and perfluoropropionates and mixtures thereof.

The mesh materials for controlled release of antivirals of the present technology can be used as systems for the controlled release of antimicrobial and antiviral compounds, including against coronaviruses, and can be applied, without limitation, in air conditioning filters, disposable materials, personal protective equipment, packaging, clothes.

The present technology can be better understood based on the examples presented below, without being limiting.

The present technology is characterized by the combination of materials with a fibrous structure, comprising a mixture of at least one different material, in which at least one of the materials comprises a filament, such as a polypropylene filament, and at least one other different material, from the first material, comprises a solid additive such as a fiber and/or a particulate.

Among the materials of fibrous structures that can be used as solid additives are wood pulp fibers and/or absorbent gel materials and/or filler particles and/or spot bonding powders of particles and/or clays and filaments, such as polypropylene filaments; wood pulp fibers, rayon, which in turn includes but is not limited to viscose, lyocell, cotton, wool, silk, jute, flax, ramie fibers, hemp, flax, camel hair, kenaf; synthetic staple fibers made from polyester, nylon, polyolefins such as polypropylene; natural polymers such as starch, starch derivatives, cellulose and cellulose derivatives, hemicellulose, hemicellulose derivatives, chitin, chitosan, polyisoprene (cis and trans), peptides, polyhydroxyalkanoates, polyolefin copolymers such as polyethylene-octene and biodegradable or compostable thermoplastic fibers, such as polylactic acid filaments, polyvinyl alcohol filaments and polycaprolactone filaments based on polymers such as polyethylene (PE), low density polyethylene (LDPE), high density polyethylene (HDPE), polypropylene (PP), ethyl vinyl acetate (EVA) or based on biodegradable polymers such as poly(lactic acid-co-glycolic acid) (PLGA), polylactic acid (PLA) and mixtures thereof, among others.

Among the filament structures there are included meltblown and/or spun filaments, such as starch and its derivatives, cellulose derivatives and cellulose, hemicellulose and derivatives, chitin, chitosan, polyisoprene, peptides, polyhydroxyalkanoates and synthetic polymers including among others thermoplastic polymer filaments comprising thermoplastic polymers such as polyesters, nylons, polyolefins, such as polypropylene filaments, polyethylene filaments, polyvinyl alcohol derivatives and polyvinyl alcohol, sodium polyacrylate filaments and polyolefin copolymers, such as polyethyleneproctene and biodegradable or compostable thermoplastic fibers such as polylactic acid filaments, polyvinyl alcohol filaments and polycaprolactone filaments.

### EXAMPLE 1 - PREPARATION OF MESH MATERIALS WITH CHLORHEXIDINE:CYCLODEXTRIN INCLUSION COMPOUNDS.

In this example, the impregnation of the mesh matrices with the inclusion compounds of chlorhexidine: beta-cyclodextrin in the molar ratio 1:1, 1:2, 1:3 and/or 1:4 was performed. The groups containing the fabrics as specimens 2720, 2774 and 2990 were prepared carrying: a. 5% Chlorhexidine, b. containing 5% chlorhexidine + 1% starch, and c. 2720 carrying chlorhexidine:cyclodextrin compound. In all cases, the specimen was placed in contact for adsorption of the above-mentioned materials, for one hour, and then dried in an oven at a temperature of 100 °C.

Controlled release systems of fabrics were prepared using the inclusion compounds of chlorhexidine:cyclodextrins using the Foulard method (soaking, squeezing and fixing). Specimens of 35 × 35 cm² were made of 4 different types of fabric, references TX20G11 (grammage 299.52 g/m²), TX20G21 (grammage 322.56 g/m²), TX20G31 (grammage 156.00 g/m²) and TX20G41 (grammage 165.99 g/m²).

The preparation of mesh materials used cellulose (paper, as substrate for masking tape) and non-woven fabric (NWF) as a matrix for the inclusion compounds of chlorhexidine:cyclodextrin. The use of Teflon varnish in water (diluted 2%) and water-based acrylic varnish on masking tape was also tested, with an immersion time of the NWF matrices or masking tape of at least 5 seconds. The excess of varnish with the inclusion compound was removed by a pressing system, composed of cylinders. Some samples underwent a drying process with temperature and others with ultraviolet rays (UV rays), simulating the large-scale production processes of adhesive tapes.

The mesh materials containing free chlorhexidine or chlorhexidine:cyclodextrin inclusion compound showed virucidal activity, but at different contact times. Chlorhexidine 5.0% inactivated 99.99% of the coronavirus (MHV-3 Strain) in contact times of 1 and 5 minutes and inhibited 99.9% in contact time of 10 minutes. The compound chlorhexidine:beta-cyclodextrin 5% (1:2) inactivated up to 99.9999% the coronavirus (MHV-3 Strain) at all tested times (up to 4 hours).

The mesh materials proved to be able to inhibit viral infection, as the encapsulated product containing chlorhexidine:cyclodextrin 5% (1:2 molar ratio) was effective for the inactivation/destruction of viral particles, thus being a virucidal agent for the Coronavirus group.

The inclusion compound showed efficacy of up to 1,000 times compared to unencapsulated chlorhexidine. These results are surprising for chlorhexidine and for the chlorhexidine:cyclodextrin inclusion compound when compared to the state of the art, which shows that chlorhexidine has a very weak antiviral effect or is inactive.

The technology also reports the surprising effect of the antiviral activity and durability test, "long lasting", of the chlorhexidine:cyclodextrin inclusion compound (chemical compound applied to a textile substrate) which was able to inactivate the Coronavirus for up to ten (30) days at a concentration of 1.25% (m/v), when compared with free chlorhexidine at a concentration of 5%, which lost its activity after 10 minutes. Cytotoxicity Tests demonstrated that the mixture of chlorhexidine:cyclodextrin inclusion compound in fabrics was not cytotoxic to the tested cell line: NCTC clone 929 L cell (ATCC^{®} CCL-1 ^{™}).

### EXAMPLE 2 - OBTAINING THE INCLUSION COMPOUNDS USING THE LYOPHILIZATION METHOD.

Two batches of inclusion compounds were obtained: Batch #1: 80.43565 g of beta-cyclodextrin were weighed, which were dissolved in 450 mL of distilled water. To facilitate the process, the mixture was stirred and heated to a temperature of 80 °C. Following solubilization, 147 mL of 20% (m/v) chlorhexidine gluconate aqueous solution were added slowly.

Batch #2: 182.8140 g of beta-cyclodextrin were weighed, which were dissolved in 1000 mL of distilled water. To facilitate the process, the mixture was stirred and heated to a temperature of 80 °C. Following solubilization, 333 mL of 20% (m/v) chlorhexidine gluconate aqueous solution were slowly added.

Both batches #1 and #2 were packaged in glassware suitable for lyophilization. They were frozen using liquid nitrogen and placed to dry using the lyophilization method for 72 h. A low-density white powder solid was obtained.

### EXAMPLE 3 - OBTAINING THE INCLUSION COMPOUNDS USING THE SPRAY-DRYER METHOD.

Batch #3: 205.7934 g of beta-cyclodextrin were weighed, which were dissolved in 1125 mL of distilled water. To facilitate the process, the mixture was stirred and heated to a temperature of 80 °C. After solubilization, 376 mL of 20% (m/v) chlorhexidine gluconate aqueous solution were slowly added under constant stirring and heating. The mixture was fed to the Mini Spray Dryer, Trademark Buchi B290, using the following experimental parameters for drying the inclusion compound: Inlet temperature: 140 °C, Aspirator: 90%, Pump: 49%, Outlet temperature: 62 °C, Airflow: 30 - 20.

A yellowish white and low-density powder solid was obtained. The weight after production of the inclusion compound was 207.0835 g, resulting in a yield of 78.0%.

### EXAMPLE 4 - OBTAINMENT OF CONTROLLED RELEASE SYSTEMS THROUGH THE IMPREGNATION OF MESH MATERIALS, SUCH AS FABRICS, WITH THE CHLORHEXIDINE:BETA-CYCLODEXTRIN INCLUSION COMPOUNDS.

Test specimens were prepared for analysis of anti-Covid-2 antiviral properties. 1 × 3 cm² specimens were made of five different types of fabric: references 2720 (grammage 290 g/m²), 2724 (grammage 226 g/m²), 2900 (grammage 176 g/m²), and 0001 and 0002 fabrics.

**Table 1. Details of used fabrics**

| Reference | Resource |
|---|---|
| 2720 | Cotton |
| | Green color |
| | Soft touch texture |
| | Greater thickness |
| 2724 | Gray color |
| | Rough texture |
| | Less thickness |
| 2900 | Black color |
| | Synthetic, polymeric material |
| | Elastic and porous |
| 0001 | Orange |
| | Rough texture |
| | Greater thickness |
| | Rigid |
| 0002 | White |
| | Synthetic |
| | Apparently hydrophobic |
| | Less thickness |

Group 1. The group contains 2720 specimens carrying: a. 5% chlorhexidine, b. containing 5% chlorhexidine + 1% starch, and c. 2720 carrying chlorhexidine:beta-cyclodextrin inclusion compound. In all cases, the specimen was placed in contact for adsorption of the materials for one hour. Afterwards, they were dried in an oven at a temperature of 100 °C.

Group 2. The group contains 2724 specimens carrying: a. 5% chlorhexidine, b. containing 5% chlorhexidine + 1% starch, and c. 2724 carrying chlorhexidine:beta-cyclodextrin inclusion compound. In all cases, the specimen was placed in contact for adsorption of the materials for one hour; soon after, they were dried in an oven at a temperature of 100 °C.

Group 3. The group contains 2900 specimens carrying: a. 5% chlorhexidine, b. containing 5% chlorhexidine + 1% starch, and c. 2900 carrying chlorhexidine:beta-cyclodextrin inclusion compound. In all cases, the specimen was placed in contact for adsorption of the above-mentioned materials, for one hour; soon after, they were dried in an oven at a temperature of 100 °C.

Group 4. The group contains 0001 specimens carrying: a. 5% chlorhexidine, b. chlorhexidine:beta-cyclodextrin inclusion compound, and c. 0001 fabric carrying chlorhexidine:beta-cyclodextrin inclusion compound. In all cases, the specimen was placed in contact for adsorption of the aforementioned materials for one hour, after which they were dried in an oven at a temperature of 100 °C.

Group 5. The group contains 0002 specimens carrying: a. 5% chlorhexidine, b. chlorhexidine: hydroxypropyl-beta-cyclodextrin inclusion compound, and c. 0002 fabric carrying chlorhexidine:beta-cyclodextrin inclusion compound. In all cases, the specimen was placed in contact for adsorption of the materials for one hour. Then, they were dried in an oven at a temperature of 100 °C.

### EXAMPLE 5 - PREPARATION OF FABRIC CONTROLLED RELEASE SYSTEMS USING THE CHLORHEXIDINE:CYCLODEXTRIN INCLUSION COMPOUNDS USING THE FOULARD METHOD (SOAKING, SQUEEZING AND FIXING).

Specimens of 35 × 35 cm² of 4 different types of fabric were made, references TX20G11 (grammage 299.52 g/m²), TX20G21 (grammage 322.56 g/m²), TX20G31 (grammage 156.00 g/m²) and TX20G41 (grammage 165.99 g/m²).

Group 1. The group contains the TX20G11, TX20G12 and TX20G13 specimens carrying, respectively: 5.0 g L⁻¹; 7.5 g L⁻¹ and 10 g L⁻¹ of chlorhexidine:beta-cyclodextrin inclusion compounds.

Group 2. The group contains the TX20G21, TX20G22 and TX20G23 specimens carrying, respectively, 5.0 g L⁻¹; 7.5 g L⁻¹ and 10 g L⁻¹ of chlorhexidine:beta-cyclodextrin inclusion compounds.

Group 3. The group contains the TX20G31, TX20G32 and TX20G33 specimens carrying, respectively, 5.0 g L⁻¹; 7.5 g L⁻¹ and 10 g L⁻¹ of chlorhexidine:beta-cyclodextrin inclusion compounds.

Group 4. The group contains the TX20G41, TX20G42 and TX20G43 specimens carrying, respectively: 5.0 g L⁻¹; 7.5 g L⁻¹ and 10 g L⁻¹ of chlorhexidine:beta-cyclodextrin inclusion compounds.

To prepare the baths, the amount needed for the three concentrations described above was weighed. The solute was added in water at room temperature and the solution was taken to the mixer, leaving it there for 3 min at 800 rad.s⁻¹; the substrate was soaked in the Foulard, with a residence time in the immersion tank equal to 4 seconds. Drying in a thermosetting device at a temperature of 160 °C, with a residence time of 90 seconds for samples from groups TX20G11 and TX20G21, and 60 seconds for samples from groups TX20G31 and TX20G41. There follows Table 2, which shows the pick-up and mass change of the product after application:

**Table 2. Consumption, in mass (g), of product per article**

| n | Color | Ideal g L⁻¹ | Pickup (%) | Actual g L⁻¹ | g m⁻¹ | g m⁻² |
|---|---|---|---|---|---|---|
| 1 | Black | 5.0 | 60 | 3.0 | 0.8986 | 0.6240 |
| | | 7.5 | | 4.5 | 1.3478 | 0.9360 |
| | | 10.0 | | 6.0 | 1.7971 | 1.2480 |
| 2 | Beige | 5.0 | 65 | 3.3 | 1.0483 | 0.7280 |
| | | 7.5 | | 4.9 | 1.5725 | 1.0920 |
| | | 10.0 | | 6.5 | 2.0966 | 1.4560 |
| 3 | Cream | 5.0 | 65 | 3.3 | 0.5070 | 0.3250 |
| | | 7.5 | | 4.9 | 0.7605 | 0.4875 |
| | | 10.0 | | 6.5 | 1.0140 | 0.6500 |
| 4 | White | 5.0 | 80 | 4.0 | 0.6636 | 0.4200 |
| | | 7.5 | | 6.0 | 0.9954 | 0.6300 |
| | | 10.0 | | 8.0 | 1.3272 | 0.8400 |

**Table 3. Linear grammage and substrate yields**

| | Color | Linear Grammage(g m⁻¹) | Yield (m kg⁻¹) | Pickup (%) |
|---|---|---|---|---|
| 1 | Black | 299.52 | 3.34 | 65 |
| 2 | Beige | 322.56 | 3.10 | 65 |
| 3 | Cream | 156.00 | 6.41 | 65 |
| 4 | White | 165.90 | 6.03 | 80 |

### EXAMPLE 6 - OBTAINING A MESH MATERIAL BASED ON CELLULOSE (PAPER), FABRIC AND NON-WOVEN FABRIC (NWF) AS MATRIX FOR THE CONTROLLED RELEASE OF CHLORHEXIDINE:BETA-CYCLODEXTRIN.

3 groups of specimens were created, totaling 22 samples. The tested bases received different combinations of varnish, adhesives, infrared process and the chlorhexidine:beta-cyclodextrin inclusion compound. In all samples, we tried to simulate the scale production equipment.

For the test specimens developed, Teflon Varnish (NWF) was used, being Teflon in water (diluted 2%) or acrylic polymer varnish in water (diluted 2%). The immersion time of the matrices (NWF or masking tape) was 5 seconds, simulating the production process on a scale. Excess varnish with the chlorhexidine:beta-cyclodextrins inclusion compound was removed using two glass rods together, simulating two cylinders pressing the matrix of interest (NWF or masking tape).

NWF sample group: The NWF process has 3 important steps for the insertion of the inclusion compound chlorhexidine:beta-cyclodextrin: i) passage through the varnish (Teflon diluted in water); ii) passage of the adhesive; iii) cut. Another important step is curing the varnish, which goes through an infrared (IR) chamber. In this IR process, the material (NWF + Varnish + chlorhexidine:beta-cyclodextrin inclusion compound) reaches a temperature of around 120 °C, with an exposure time of around 20 seconds.

AD20F08, AD20F12, AD20F15 and AD20F16 samples, whose results are shown in Table 6, followed similar processes, with variations in concentration and passage through the IR process. As an example, the AD20F16 sample followed the following procedure: 705 mg of chlorhexidine:beta-cyclodextrin inclusion compound were added in a 1:2 molar ratio, as claimed in the present technology, at a concentration of 4% (m/v) to 75 g varnish (ready Teflon diluted in water). The NWF was immersed for about 2 to 3 seconds in the varnish + inclusion compound solution (in scale, the production time is 20 m/min). The excess is removed with the help of two glass sticks (simulating the compression rollers of the industrial process), subsequently dried in an oven at 120 °C. After drying, the NWF received the MOPP adhesive, in the amount of 44 g/m². The incorporated measurements, in mass, of varnish and varnish + inclusion compound were, respectively: 52.4 g/m² and 54.2 g/m². The white NWF sample had an initial weight of 48.9 g/m².

Cellulose-based sample group (crepe paper): The masking tape production process has 4 important steps for the insertion of chlorhexidine:beta-cyclodextrin: i) saturation; ii) varnish; iii) adhesive; iv) cut. In the case of masking tape, there is no infrared curing process.

In the AD20F22 sample, which was effective after 12 hours: the crepe paper (40 g/m²) already had the removable adhesive applied. The chlorhexidine:beta-cyclodextrin inclusion compound was added in molar ratios 1:1 and 1:2, at a concentration of 4% (m/v), in the water-based varnish solution. The functionalization process was carried out by dripping and subsequent spreading with a steel rod with grooves. The final absorption of the inclusion compound in the dry sample was approximately 1 g/m².

### EXAMPLE 7 - PROTOCOL FOR INSERTION AND RELEASE OF THE CHLORHEXIDINE ACTIVE PRINCIPLE IN DIFFERENT MATRIXES: NWF, FABRICS AND BASED-ON-CELLULOSE MATERIALS.

The procedure was adapted from the USP 26 (2003) standard. Shaking tray: temperature 25 °C, speed (50 - 100 rpm). Samples of each matrix based on mesh materials with a dimension of 10 × 10 mm, in triplicate, were placed in an appropriate container and there were added 3 mL of water with chlorhexidine release medium and with the chlorhexidine:beta-cyclodextrin inclusion compounds. The release process consists of the following steps: a) 3 mL of water were added to each container; b) 3 mL aliquots were taken every hour during the first eight hours, then every 24 hours until the end of the test for up to 7 days. After removing the aliquots, a new release medium (3 mL of water) was added. Chlorhexidine was quantified using absorption techniques in the ultraviolet-visible region (UV-Vis) and ultra-performance liquid chromatography (UPLC), methods known in the state of the art for the quantification of this type of active principle.

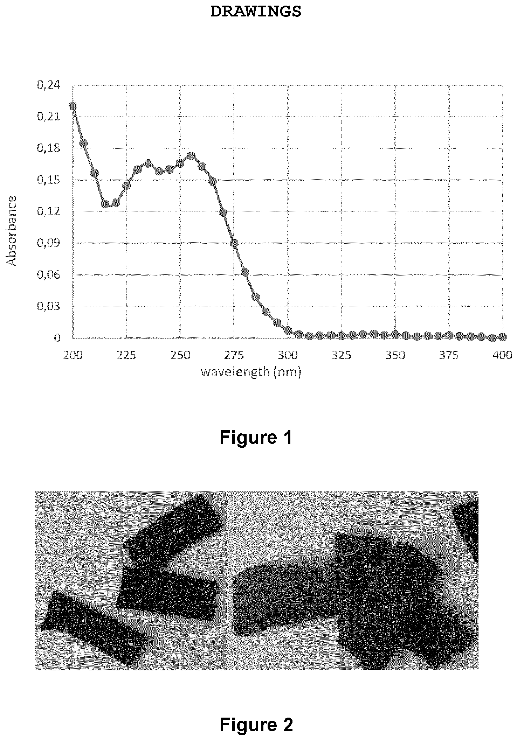

Figure 1 shows two maximums referring to the release of chlorhexidine in the ultraviolet-visible region, in aqueous medium.

### EXAMPLE 8 - PROTOCOL FOR THE WASHING TEST PROTOCOL OF FABRIC WITH NON-IONIC DETERGENTS AND WASHING RESISTANCE.

Procedure adapted from technical standards NTC 1155-2 and ISO 105C06. Mechanical washing device: it consists of a water bath that contains a rotating shaft, which radially supports containers of 75 mm in diameter, 125 mm in height and capacity of 550 mL, and the bottom of the containers is 45 mm from the center of the shaft. The shaft coupling is rotated at a frequency of 40 rpm. The temperature of the water bath is thermostatically controlled to keep the test solution at the set temperature. Detergent solution: dissolve 4 g of detergent per liter of solution. Prepare a minimum volume of 1 liter of solution, due to the possible lack of homogeneity of the detergent.

Sample: A fabric sample is mounted with 100 mm by 40 mm. The test conditions were: temperature 40 °C, wash bath volume 300 mL, time 45 min. Procedure: add the volume of wash solution to each test container, adjust the temperature of the wash bath, add the fabric sample to the container, close the container, turn on the device. At the end of the test time, the samples were removed and rinsed twice, for 1 minute each time, in two different baths of 100 mL of water at 40 °C, the excess water in the sample was removed and the fabric was dried by exposure on the air. Figure 2 shows the fabrics after washing with detergent.

### EXAMPLE 9 - TESTS OF THE ANTIVIRAL ACTIVITY OF MESH MATERIALS AS CHLORHEXIDINE AND CHLORHEXIDINE:BETA-CYCLODEXTRIN CONTROLLED RELEASE SYSTEMS.

Table 4 shows the results of the virucidal test performed for the initial fabrics 270, 2724 and 2900. The samples impregnated with 2-hydroxypropyl-beta-cyclodextrin (HP-BCD) 5% did not inhibit the viral infection, that is, have not been activated for MHV3 Coronavirus. Samples impregnated with inclusion compound of HP-BCD:Clorhexidine and BCD:chlorhexidine were able to inhibit 100% of the replication of the MHV3 Coronavirus, whereas sample 08 (HP-BCD + Clr 5% + 002 fabric) inhibited 99.99% with "viral ineffectiveness reduction" of ≥ log 4; therefore, in this group of samples, all were virucidal.

**Table 4. Virucidal activity results for the HP-BCD 5%, HP-BCD + chlorhexidine 5% and BCD + chlorhexidine 5% systems**

| **Sample Number** | **Description** | **Toxicity** | **Viral Inactivation** | **Viral Titer** |
|---|---|---|---|---|
| 1 | HP-BCD 5% | 1:10 | Not inactivated | 10⁸ / mL |
| | Fabric 2720 | | | |
| 2 | HP-BCD 5% | Not cytotoxic | Not inactivated | 10⁸ / mL |
| | Fabric 2724 | | | |
| 2.1 | HP-BCD 5% | Not cytotoxic | Not inactivated | 10⁸ / mL |
| | Fabric 2724 | | | |
| | Residue *A.I. | | | |
| 3 | HP-BCD 5% | Not cytotoxic | Not inactivated | 10⁸ / mL |
| | Fabric 2900 | | | |
| 4 | HP-BCD 5% | Not cytotoxic | Not inactivated | 10⁸ / mL |
| | Fabric 0001 | | | |
| 5 | HP-BCD 5% | Not cytotoxic | Not inactivated | 10⁸ / mL |
| | Fabric 0002 | | | |
| 6 | HP-BCD +Clr 5% | Up to 1:100 (10²) | Inactive | 0.0 |
| | Fabric 2724 | | | |
| 6.1 | HP-BCD +Clr 5% | Up to 1:100 (10²) | Inactive | 0.0 |
| | Fabric 2724 | | | |
| | Residue *A.I. | | | |
| 7 | HP-BCD +Clr 5% | Up to 1:100 (10²) | Inactivated | 0.0 |
| | Fabric 2900 | | | |
| 8 | HP-BCD +Clr 5% | Up to 1:100 (10²) | Inactivated | 10³ / mL |
| | Fabric 0002 | | | |
| 9 | BCD +Clr 5% | Up to 1:100 (10²) | Inactivated | 0.0 |
| | Fabric 2724 | | | |
| 10 | BCD +Clr 5% | Up to 1:100 (10²) | Inactivated | 0.0 |
| | Fabric 2900 | | | |
| 11 | BCD +Clr 5% | Up to 1:100 (10²) | Inactivated | 0.0 |
| | Fabric 0002 | | | |

| | | | | |
|---|---|---|---|---|
| *A.I. - after impregnation | | | | |

Table 5 presents the results of the virucidal test performed for 2720, 2724, 2900, 0001 and 0002 fabrics. Fabrics impregnated with 5% chlorhexidine showed antiviral activity of 99.99%.

**Table 5. Results of 5% chlorhexidine and 5% BCD virucidal activity**

| **Sample Number** | **Description** | **Time (h)** | **MHV-3 Coronavirus Activity** | **Percent of inactivation** | **Activity** |
|---|---|---|---|---|---|
| 1 | Clr 5% | 1 | 99.99% Virucidal Activity | 1 h - 1:10³ (99.99%) | Virucidal |
| | Fabric 2720 | | | | |
| 2 | Clr 5% | 1 | 99.99% Virucidal Activity | 1 h - 1:10³ (99.99%) | Virucidal |
| | Fabric 2724 | | | | |
| 3 | Clr 5% | 1 | 99.99% Virucidal Activity | 1 h - 1:10³ (99.99%) | Virucidal |
| | Fabric 2900 | | | | |
| 4 | Clr 5% | 1 | 99.99% Virucidal Activity | 1 h - 1:10³ (99.99%) | Virucidal |
| | Fabric 0001 | | | | |
| 5 | Clr 5% | 1 | 99.99% Virucidal Activity | 1 h - 1:10³ (99.99%) | Virucidal |
| | Fabric 0002 | | | | |
| 6 | BCD 5% | 1 | 99.99% Virucidal Activity | 1 h - 1:10³ (99.99%) | Virucidal |
| | Fabric 2724 | | | | |
| 7 | BCD 5% | 1 | Not effective | 1 h - Not effective | |
| | Fabric 2900 | | | | |
| 8 | BCD 5% | 1 | 80.00% virucidal activity | 1 h - 1:10⁷ (90%) | |
| | Fabric 0002 | | | | |

Table 6 presents the results of virucidal activity of NWF and fabric impregnated with inclusion compound. All samples were oven dried at 120 °C for 5 minutes.

**Table 6. Composite virucidal activity results of inclusion in FABRIC and NWF**

| **Sample** | **Substrat e** | **Inclusion compoun d diluted in** | **Inclusion compound concentrati on** | **Tests** | **Test time** | **Inhibi tion (%)** | **Test time** | **Inhibiti on (%)** |
|---|---|---|---|---|---|---|---|---|
| AD20F08 | Fabric | Varnis h Teflon (NWF) | 2% | Corona Virus MHV-3 Strain | 5 min | 99.9 0 | 2 hours | 99.90 |
| AD20F12* | NWF | Teflon Varnish (NWF) | 2% | Corona Virus MHV-3 Strain | 5 min | 99.9 0 | 2 hours | 99.90 |
| AD20F15 ** | NWF | Teflon Varnis h (NWF) | 2% | Coron a Virus MHV-3 Strain | 5 min | 99.90 | 2 hours | 99.90 |
| AD20F16 * | NWF | Teflon Varnis h | 4% | Coron a Virus MHV-3 Strain | 5 min | 99.9 0 | 2 hours | 99.99 |

| | | | | | | | | |
|---|---|---|---|---|---|---|---|---|
| *Sample has gone through IR process "Sample has not gone through the IR process | | | | | | | | |

The tested virus was the Coronavirus MHV-3 Strain genus Betacoronavirus (same genus and family of species SARS-CoV-1, SARS-CoV-2, MERS and others). Viruses Cell Lines, Coronavirus MHV-03 cell: NCTC clone 929 L cell (ATCC^{®} CCL-1 ^{™}).

The tests were carried out in a NB-2 laboratory (Biosafety Level 2) following the Recommendations of ANVISA, Art. 1 and Art. 3 of IN 04/13 and IN 12/16 and methodologies described in the standards (EN14476:2019, ASTM E1053 -11 and the Robert Koch Institute - RKI) and following the "Good Laboratory Practices" (GLP). As the culture medium for viruses and cell lines there was used Dulbecco's Minimal Essential Medium (DMEM) containing 10% fetal bovine serum.

The titration of the Coronaviruses (MHV-3 Strain) was performed according to the DICT50 method (Fabric Culture Infectious Doses 50%). Sequential base 10 virus dilutions were performed in quadruplicate in sterile 96-well microplates. Next, L929 cells were added at a concentration of 2 × 10⁵ cells/well. After 48 hours, the cytopathic effect (CPE) of the viral infection is verified, in comparison with cell control and viral control.

Samples of 5% cyclodextrin and 5% cyclodextrin:beta-cyclodextrin (1:2) were diluted in DMEM and individually treated. The two products were individually mixed with the virus and subsequently subjected to different contact times (immediate, 1 minute, 5 minutes, 10 minutes, 1 hour and 4 hours). Finally, they were inoculated to the permissive cell (L929 - mouse fibroblast cell), with 2 × 10⁵ cells/well.

In the microplates of 96 wells, the products and the virus were added. Cell systems were incubated at 37 °C in an oven with 5% CO₂ for 48 h. Titers were calculated based on the method of Reed and Muench, 1938.

Results are expressed as percent viral inactivation compared to untreated viral control (virus titer). Negative: cell control (2 × 10⁵ cells/mL) in DMEM medium, without virus and without test sample; Virus control: Virus titration (101 to 1012) and cell culture in DMEM medium.

The results are shown in Table 7 of the inactivation of the Coronavirus (MHV-3 Strain) in relation to the products 5% chlorhexidine and the 5% chlorhexidine:cyclodextrin inclusion compound (1:2) and contact times:

**Table 7. Result of the inactivation of the Coronavirus (MHV-3 Strain)**

| Products | Contact Times | Infectivity Reduction (Virus Titer 10⁸/mL) | Coronavirus (MHV-3 Strain) Table 3* |
|---|---|---|---|
| 5% Chlorhexidine | Immediate | - | Not Effective |
| | 1 minute | 10⁴ | *99.99% (virucidal) |
| | 5 minutes | 10⁴ | 99.99% (virucidal) |
| | 10 minutes | 10⁵ | 99.99% |
| | 1 hour | - | Not effective |
| | 4 hours | - | Not effective |
| 5% Cyclodextrin: chlorhexidine (2:1) encapsulated | Immediate | 10⁴ | 99.99% (virucidal) |
| | 1 minute | 10³ | 99.99% (virucidal) |
| | 5 minutes | 10² | 99.999% (virucidal) |
| | 10 minutes | 10² | 99.9999% (virucidal) |
| | 1 hour | 10² | 99.9999% (virucidal) |
| | 4 hours | 10² | 99.9999% (virucidal) |

Table 8 shows the comparison between 5% chlorhexidine and 5% cyclodextrin:chlorhexidine (2:1) by contact times in relation to virucidal activity.

**Table 8. Result comparing virucidal activity**

| Reduced Infectivity Coronavirus (MHV-3 Strain) - Virus Titer 10⁸/mL | | | |
|---|---|---|---|
| Contact times | 5% Chlorhexidine | 5 % Chlorhexidine:cyclodextrin (1:2) | Result |
| 1 minute | 10⁴ | 10³ | 10 times more effective |
| 5 minutes | 10⁴ | 10² | 100 times more effective |
| 10 minutes | 10⁵ | 10² | 1000 times more effective |

Thus, it is ascertained that both products showed virucidal activity, but at different contact times: 5% Chlorhexidine - Inactivated 99.99% of the Coronavirus (MHV-3 Strain) in the contact times of 1 and 5 minutes and inhibited 99.9% in the contact time of 10 minutes. 5% Chlorhexidine:beta-cyclodextrin (1:2) - Inactivated up to 99.9999% of the Coronavirus (MHV-3 Strain) at all tested times (up to 4 hours).

Considering that there was inhibition of viral infection, it can be concluded that the product 5% chlorhexidine:beta-cyclodextrin (1:2) was effective for the inactivation/destruction of viral particles, and therefore, there is recommended the use as a potential virucidal agent for the Coronavirus group and in the contact times tested. The encapsulated product (chlorhexidine:beta-cyclodextrin) was 10, 100 and 1000 times more effective compared to the non-encapsulated one.

Fabrics (cotton and synthetic) were sterilized and cut to a size of 1 cm² where the chlorhexidine:beta-cyclodextrin compound was applied to 30 pieces of fabric. Tests were performed in quadruplicate (4 biological replicates).

Positive control of the cell line: presence of the virus, fabric with addition of the test product and cell system; control of cell toxicity: fabric with addition of encapsulated compound and cell line; durability control: the fabric contact time with the addition of chlorhexidine:beta-cyclodextrin compound was 10 days at room temperature and in closed sterile tubes; negative cell line control: cell system only, without the presence of viruses and without the presence of fabrics with disinfectants; virus control: virus titration in cell culture.

The insertion of the virus to the chlorhexidine:beta-cyclodextrin compound in fabrics was subjected to different dilutions and times (1, 2, 5, and 10 minutes). To test the durability of the product on fabrics there were carried out tests after 10 days of contact, showing antiviral activity of materials impregnated with the antiviral inclusion compounds claimed in the present technology in up to 10 days.

Plates with chlorhexidine:beta-cyclodextrin compound in fabrics + Virus + cell system were incubated at 37 °C in an oven with 5% CO₂ for 48 h for 3 days. The virus titer was expressed as log10 TCID50/mL from the calculation performed following the Spearman & Karber method (Lorenz & Bögel, 1973). The evaluation was visual through reading in an inverted microscope looking for the occurrence of the Cytopathic Effect (CPE) characteristic of the coronavirus.

The result is considered negative if ECP has occurred and positive when EPC is not present, that is, the test substance inhibited the viral action on the cell (Omonike, *et al.,* 2018).

The virucidal activity of the procedure is determined from the logarithmic difference in viability between treated and control carriers.

The infection/contamination of different fabrics for Coronavirus (MHV-3 Strain) was inhibited for the chlorhexidine:beta-cyclodextrin compound when applied at a concentration of 1.25% (m/v) for the contact time from 2 minutes and up to 10 days of contact.

In this way, the durability test of the chlorhexidine:beta-cyclodextrin compound was observed in the inactivation of the Coronavirus in up to ten (10) days at a concentration of 1.25% (m/v).

Cytotoxicity tests showed that the chlorhexidine:beta-cyclodextrin mixture in fabrics + viruses + cell system was not toxic to the tested cell line; therefore, they did not cause damage to living cells at a concentration of 1.25% (m/v).

Considering that there was inhibition of viral infection, it can be concluded that the chlorhexidine:beta-cyclodextrin compound was effective for the inactivation/destruction of viral particles in fabrics, and, therefore, we recommend its use at a concentration of 1.25% (m/v) as a potential virucidal agent in fabrics for all viruses of the Coronavirus group for a contact time from 02 minutes to up to 30 days.

### EXAMPLE 10 - FIELD TEST: INHIBITION HALO, ESCHERICHIA COLI BACTERIUM.

The study of antimicrobial activity against the *Escherichia coli* bacterium (ATCC 25922) was carried out following the ANVISA protocol for the study of sensitivity to antimicrobials by disc-diffusion in agar (Standardization of antimicrobial selectivity tests by disc-diffusion: approved Standard. 8th ed.).

In table 9, there are the results of the strips containing the inclusion compound inserted in the strips used in 7, 14, 21, and 28 days against the *E*. *coli* bacteria.

**Table 9. Result of the antimicrobial activity of the strips against E.coli bacteria**

| Point of application | 07 days | 14 days | 21 days | 28 days |
|---|---|---|---|---|
| Cold chamber table - TECA | Active | Active | Active | Active |
| Escalator - PA | Active | Active | No Collection | No Collection |
| Check-in counter - PA | Active | Active | Active | Active |
| Chairs - ADM | Active | Active | No Collection | Active |
| Inspection Table of IRS 8L | Active | Active | Active | Active |
| Elevator ADM | Active | Active | Active | Active |
| Piazza Table Passenger Terminal - PT | No Collection | Active | No Collection | Active |
| Female Toilet Passenger Terminal - PT | No Collection | Active | No Collection | Active |
| Piazza Table Passenger Terminal - PT | No Collection | Active | No Collection | Active |
| Strip without chlorhexidine: beta-cyclodextrin | Inactive | Inactive | Inactive | Inactive |
| Strip with chlorhexidine: beta-cyclodextrin | Active | Active | Active | Active |

The strips referring to the locations Escalator - PA, Chairs - ADM, Piazza Table passenger terminal - PT and Female toilet passenger terminal - PT were not evaluated in the 3^{rd} week (21 days). The 28-day reading for Chairs - ADM and Piazza Table passenger terminal - TPI strips showed antimicrobial activity. The controls used in the experiments were the strip without and strip with the chlorhexidine:beta-cyclodextrin inclusion compound (1:2).It was observed that only the strip with the inclusion compound showed antimicrobial activity in all experiments performed.

It is concluded that the studies of the antimicrobial action of the strips containing the inclusion compounds showed antibacterial activity against the *Gram*-negative bacteria *Escherichia coli* within 28 days of use. The strips evaluated up to 14 days showed continuous activity after collection. The strips collected in diverse periods until the analysis of their last collection were active against the microorganism. The strip containing the inclusion compound not used in the analysis space remained with antimicrobial activity after the 28 days of testing.

### EXAMPLE 11 - PAPER AND NWF TAPE TEST AGAINST SARS-COV-2.

The adhesive tapes were produced on a pilot scale to study the antiviral activity. 3,500 square meters of paper (crepe) and non-woven fabric (NWF) tapes were produced on industrial machines, following the choice of varnish and adhesive that showed the best results in the samples developed on a laboratory scale.

The samples sent for analysis were: a) AD20H29, being a NWF tape, with removable adhesive, Teflon varnish and chlorhexidine:beta-cyclodextrin inclusion compound at a concentration of 4% (m/v) added to the varnish diluted in water; b) AD20H31, with cellulose-based paper tape, removable adhesive, water-based varnish and chlorhexidine:beta-cyclodextrin inclusion compound at a concentration of 4% (m/v) added to Teflon varnish.

Table 10 presents the results of the virucidal activity of the paper tape and NWF incorporated with the chlorhexidine:beta-cyclodextrin inclusion complex in the industrial process, at different contact times, against the SARS-CoV-2 virus.

**Table 10. Result of antiviral activity against the SARS-CoV-2 virus**

| **Samples** | **Test 1** | | **Test 2** | | **Contact time** |
|---|---|---|---|---|---|
| | **Virus/mL (SARS-CoV-2)** | **Inhibition (%)** | **Virus/mL (SARS-CoV-2)** | **Inhibition (%)** | |
| Positive control, virus only | 4.56 x 10⁸ | | 4.68 x 10⁸ | | 1 min |
| Positive control, masking tape without active chlorhexidine: beta-cyclodextrin | 6.91 x 10⁸ | | 2.27 x 10⁹ | | |
| Masking tape with chlorhexidine: beta-cyclodextrin active (AD20H31) | 1.27 x 10⁶ | 99.72 | 3.70 x 10⁵ | 99.92 | |
| Positive control, NWF without chlorhexidine: beta-cyclodextrin active | 6.96 x 10⁸ | | 4.75 x 10⁸ | | |
| NWF with chlorhexidine: beta-cyclodextrin active (AD20H29) | 8.34 x 10⁵ | 99.82 | 5.39 x 10⁵ | 99.88 | |
| Negative Control (used to rule out artifacts from the RT-PCR test) | Not Detected | | Not Detected | | |
| Positive control, | 4.62 x | | 8.18 x | | |
| virus only | 10⁸ | | 10⁸ | | |
| Positive control, masking tape without chlorhexidine: beta-cyclodextrin active | 7.10 x 10⁸ | | 1.85 x 10⁹ | | |
| Masking tape with the chlorhexidine: beta-cyclodextrin active (AD20H31) | 7.29 x 10⁵ | 99.84 | 2.24 x 10⁶ | 99.73 | |
| Positive control, NWF without chlorhexidine: beta-cyclodextrin active | 3.75 x 10⁸ | 18.79 | 6.38 x 10⁸ | 21.99 | 30 min |
| NWF with chlorhexidine: beta-cyclodextrin active (AD20H29) | 1.83 x 10⁵ | 99.96 | 3.44 x 10⁵ | 99.96 | |
| Negative Control (used to rule out artifacts from the RT-PCR test) | Not Detected | | Not Detected | | |
| Positive control, virus only | 2.91 x 10⁸ | | 1.71 x 10⁹ | | |
| Positive control, masking tape without chlorhexidine: beta-cyclodextrin active | 3.68 x 10⁸ | | 1.84 x 10⁹ | | |
| Masking tape with the chlorhexidine: beta-cyclodextrin active (AD20H31) | 5.73 x 10³ | 99.80 | 1.37 x 10⁶ | 99.92 | |
| Positive control, NWF without chlorhexidine: beta-cyclodextrin active | 7.65 x 10⁸ | | 1.46 x 10⁹ | 14.88 | 60 min |
| NWF with chlorhexidine: beta-cyclodextrin active (AD20H29) | 1.36 x 10⁵ | 99.95 | 2.25 x 10⁶ | 99.87 | |
| Negative Control (used to rule out artifacts from the RT-PCR test) | Not Detected | | Not Detected | | |
| Positive control, virus only | 2.30 x 10⁹ | | 1.04 x 10⁹ | | |
| Positive control, | 3.39 x | | 1.79 x | | |
| masking tape without chlorhexidine: beta-cyclodextrin active | 10⁷ | | 10⁹ | | |
| Masking tape with the chlorhexidine: beta-cyclodextrin active (AD20H31) | 2.46 x 10⁶ | 99.89 | 3.83 x 10⁵ | 99.96 | |
| Positive control, NWF without chlorhexidine: beta-cyclodextrin active | 1.77 x 10⁸ | 23.03 | 1.63 x 10⁸ | 84.32 | 120 min |
| NWF with chlorhexidine: beta-cyclodextrin active (AD20H29) | 8.69 x 10⁶ | 99.62 | 2.63 x 10⁶ | 99.75 | |
| Negative Control (used to rule out artifacts from the RT-PCR test) | Not Detected | | Not Detected | | |

### EXAMPLE 12 - LONG LASTING TEST.

The long-term tests ("Long Lasting") were performed using virus strains from the Coronavirus family (Coronavirus MHV-3). Virus (MHV-3 Strain) titration was performed according to the DICT50 method (50% Fabric Culture Infectious Doses), using sequential base 10 dilutions, performed in quadruplicate, in sterile 96-well microplates. After titration, L929 cells were added to the wells at a concentration of 2 × 10⁵ cells/well. The cytopathic effect (CPE) of the viral infection was observed after 48 hours.

3 products were tested, NWF AD20H12 (2%) incorporated by immersion bath in a solution of 2% (m/v) chlorhexidine:beta-cyclodextrin and 4% (m/v) Teflon varnish; NWF AD20H16 (4%) incorporated by immersion bath in a solution of 4% (m/v) chlorhexidine:beta-cyclodextrin and 4% (m/v) Teflon varnish; and CREPE (paper) AD20H22 (4%) incorporated by immersion bath in a solution of 2% (m/v) chlorhexidine: beta-cyclodextrin and 2% (m/v) water-based varnish. Each of the NWF, NWF (2%) and NWF (4%) products was mixed separately with the virus at different contact times, 5 minutes, 10 minutes; 2 and 4 hours; and "Long Lasting" referring to 7 days, 15 and 30 days and then inoculated to the permissive cell (L929). Microplates were incubated at 37 °C in a 5% CO₂ atmosphere and observed for cytopathic effects (CPE) daily for up to 48 hours. Titers were calculated based on the method of Reed and Muench, 1938. Results are expressed as percentage viral inactivation (Table 11) compared to untreated viral control (virus titer).

**Table 11. Essays with Coronavirus virus (MHV-3 Strain) at different times of contact with NWF incorporated to the inclusion complex**

| **Products** | **Contact times** | **Results in percentage and activity Coronavirus MHV-3** |
|---|---|---|
| NWF AD20H12 (2%) incorporated by immersion bath in a solution of 2% (m/v) chlorhexidine:beta-cyclodextrin and 4% (m/v) Teflon varnish | 5 minutes | 99.9% |
| | 10 minutes | 99.9% |
| | 2 hours | 99.9% |
| | 4 hours | 99.9% |
| | 7 days | 99.99% (virucidal) |
| | 14 days | 99.99% (virucidal) |
| | 30 days | 99.99% (virucidal) |
| NWF AD20H16 (4%) incorporated by immersion bath in a solution of 4% (m/v) chlorhexidine: beta-cyclodextrin and 4% (m/v) Teflon varnish | 5 minutes | 99.9% |
| | 10 minutes | 99.9% |
| | 2 hours | 99.9% |
| | 4 hours | 99.9% |
| | 7 days | 99.99% (virucidal) |
| | 14 days | 99.99% (virucidal) |
| CREPE PAPER AD20F22 (4%) incorporated by immersion bath in a solution of 4% (m/v) chlorhexidine: beta-cyclodextrin and 2% water-based varnish | 2 hours | 99.99% |
| | 7 days | 99.99% |
| | 14 days | 99.99% |
| | 30 days | 99.99% |

### EXAMPLE 13 - QUANTIFICATION OF CHLORHEXIDINE AND DETERMINATION OF CONTROLLED RELEASE CURVES FROM FABRIC SAMPLES AS AN EXAMPLE OF MESH MATERIALS.

The analysis of the analyte was performed using liquid chromatography coupled to a mass spectrometer, since they are the most suitable methodologies for this. In this work, an ultra-performance liquid chromatograph (UPLC) was used, initially coupled to a photodiode detector (PDA) UPLC^{®} ACQUITY and followed by a Xevo TQD mass spectrometer. The spectrometer has a triple quadrupole mass analyzer and was operated using electro-spray ionization (ESI) as the ionization source in positive mode. With these specifications, the sequential or tandem mass spectrometry technique was performed, monitoring the transitions detailed in Table 12.

**Table 12. Transitions monitored in the analysis experiment**

| **Transition** | **Cone voltage (V)** | **Collision (V)** | **Reference** |
|---|---|---|---|
| 505.3 → 336.4 | 35 | 20 | [1] |
| 505.3 → 353.1 | 35 | 20 | [1] |
| 505.0 → 170.0 | 25 | 44 | [2] |

The specifications of the spectrometer parameters are: capillary voltage: 3.00 kV, cone voltage: 102 V, extractor voltage: 3 V, radiofrequency lens voltage: 2.5 V, source temperature: 150 °C, temperature of desolvation: 450 °C, desolvation gas flow rate: 650 L/h, cone gas flow rate: 30 L/h, collision gas: argon. UPLC specifications: Column: Acquity UPLC HSS T3 (2.1 × 50 mm, 1.8 µm, Waters), column temperature: 50 °C, sampler temperature: 25 °C. Binary solvent system: solvent to deionized water with 0.1 % formic acid; B solvent methanol with 0.1% formic acid. Gradient: the total run time was 6.5 minutes using a constant flow rate of 0.45 mL/min. During the first 04 minutes, a ratio of 62% A solvent, followed by a ramp from 62% to 5% of A solvent for one minute, held for 0.5 minutes and then changed to 62% solvent in 0.01 minutes and held for one minute. Data were obtained and processed by the software Masslynx 4.1 from Waters.

The calibration curve used the following standards: Standard #1 - 99.5% chlorhexidine (CAS 55-56-1) from Sigma-Aldrich, Standard #2 - 20% solution of chlorhexidine digluconate in solution (CAS 18472-51-0). Samples with both standards were prepared in deionized water. Chlorhexidine has very low solubility in water, requiring acidification of the samples with 37% hydrochloric acid. With chlorhexidine digluconate, there were no solubility problems. The first runs with Standard #1 showed low reproducibility of the obtained areas, probably related to solubility problems. With Standard #2 it was possible to obtain a good reproducibility of the areas; therefore, it was decided to perform the calibration curve with standard #2.

In order to take into account the matrix effect, the samples of the calibration curve points were dissolved in a solution (blank) that was prepared using a sample of 2724 fabric with dimensions of 200 mm × 20 mm. The fabric was immersed in 200 mL of distilled water and placed under constant stirring. 5.0148 grams of beta-cyclodextrin was added. Stirring was continued for one hour. The fabric was removed and the solution placed in a 1000 milliliter volumetric flask, completed with deionized water. The blank was diluted to lessen the matrix effect. The runs performed only with the blank did not show a peak in the retention time of chlorhexidine, the same happened with the used mobile phase.

The preparation of the calibration curve samples, in the range of 0.1 to 2.1 ppm, was done as follows: 10 milliliters of a stock solution at a concentration of 1000 ppm in deionized water was prepared. From the stock solution, 10 milliliters of a fortification solution at a concentration of 42 ppm in deionized water was prepared. In 10 milliliter volumetric flasks, 5 mL of the blank were initially added and then the amount of fortification solution from 0.1 to 2.1 ppm. Then, the flask was made up to volume with the blank and stirred. Each curve point was prepared in quadruplicate. The solutions were transferred to chromatography vials and 5 µL were injected into the equipment according to the already-described specifications.

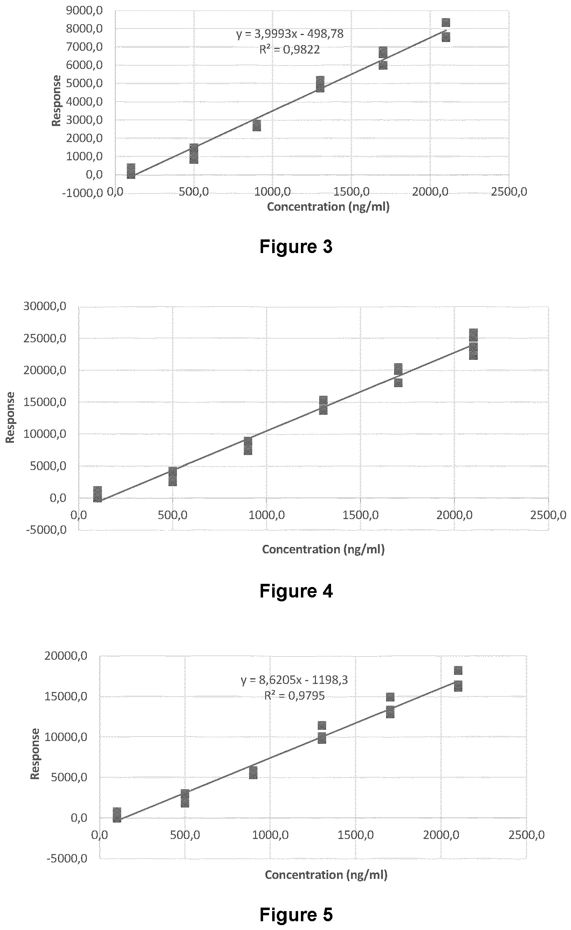

The calibration curves obtained for each channel were monitored and it was possible to detail the values of the correlation constants of the adjusted lines in addition to the adjustment value of the linear regression (Figure 3. Linear regression transition 505.3 → 353.1, Figure 4. Linear regression transition 505.3 → 336.4, and Figure 5. Linear regression transition 505.0 → 170.0).

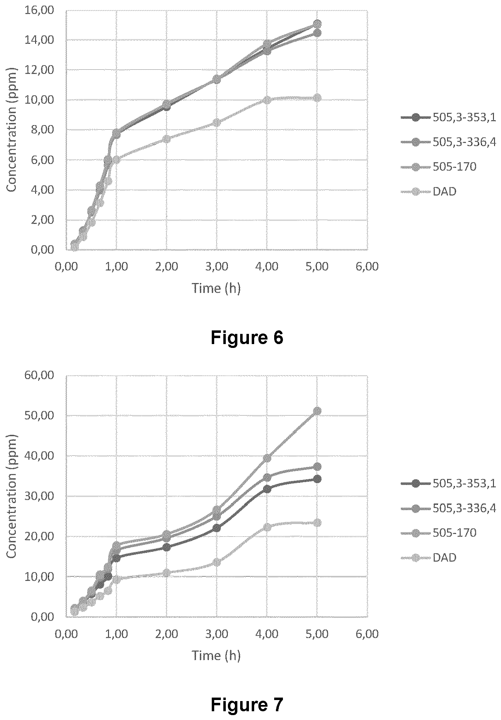

Using the calibration curves obtained, release samples of 2900 and 2724 fabrics impregnated with the inclusion compound were injected. The 2724 fabric samples needed to be diluted 5 times in deionized water since, after the first few injections, the areas were out of the analysis range. The 2900 fabric samples did not require dilution (Figure 6. 2900 fabric release curve, Figure 7. 2724 fabric release curve).

The differences in the release curves of the two fabrics show the effect of matrix, the dilution of samples from 2724 fabric decreased this effect. In this way, it is exemplified that 2774 and 2900 fabrics function as a system for the controlled release of the antiviral active principle of chlorhexidine:beta-cyclodextrins inclusion compounds.

### EXAMPLE 14 - OBTAINING CONTROLLED RELEASE SYSTEMS THROUGH THE IMPREGNATION OF MESH MATERIALS, SUCH AS POLYMERIC MATRIXES, WITH THE INCLUSION COMPOUNDS OF CHLORHEXIDINE:CYCLODEXTRIN.

There has been sought to reproduce the production process of masterbatch concentrates used in the market as additives for the functionalization of plastic films. Specimens were made using polymer bases commonly used by the sector, such as low-density polyethylene (LDPE) and ethylene vinyl acetate (EVA). A humectant was added to the base, used when working with very non-polar bases, such as polyethylene. Mineral oil was selected, which can be replaced, for example, by glycerin or sorbitan monooleate. Maleic anhydride wax and an antioxidant were also added in some specimens.

Drais-type homogenizing equipment was used, responsible for mixing the ingredients by high rotation; such a process has an average duration of 6 seconds; the generated molten mass passes through a press at 180 °C, generating 2 mm thick specimens.

The specimens were obtained from the materials described below, from which disks with an average size of 0.3 ± 0.1 cm were removed. The studies were carried out in triplicate for each material, with EVA and LDPE being the blanks, with no antimicrobial activity used in the test. *PL21E04*: EVA 73%; Betacyclodextrin 25%; Mineral oil 2%. *PL21E07*: LDPE 73%; Betacyclodextrin 25%; Mineral oil 2%. *PL21E06*: LDPE 73%; Chlorhexidine:beta-cyclodextrin (1:2) 25%; Mineral oil 2%. *PL21E10*: LDPE 68%; Chlorhexidine:beta-cyclodextrin (1:2) 25%; Mineral oil 2%; Meghwax Wax 5%. *PL21E11*: LDPE 70%; Chlorhexidine:beta-cyclodextrin (1:2) 25%; Mineral oil 2%; Antioxidant 3%.

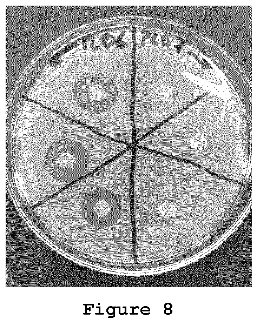

As shown in Table 13, the qualitative analyses of antimicrobial activity against the *Escherichia coli* bacterium showed that all specimens that had the chlorhexidine:beta-cyclodextrin inclusion compound (1:2) showed antimicrobial activity (Figure 8. Study of antimicrobial activity against the *Escherichia coli* bacterium by analysis of the size of the inhibition halo of PL21E06 and PL21E07 materials).

**Table 13. Relations of the amounts of polymers; compounds used and the results of inhibition zones against E.coli**

| **Sample** | **Polymer** | **L3 Inclusion compound (%)** | **β-CD 191221 (%)** | **Mineral oil (%)** | **Meghwax Wax SPP001 PP with maleic anhydride** | **Antioxidant** | **Inhibition halo against E. coli** |
|---|---|---|---|---|---|---|---|
| PL21E04 | EVA (73%) | | 25.00 | 2.00 | | | Without halo |
| PL21E06 | LDPE (73%) | 25.00 | | 2.00 | | | With halo |
| PL21E07 | LDPE (73%) | | 25.00 | 2.00 | | | Without halo |
| EVA control | EVA (98%) | | | 2.00 | | | Without halo |
| LDPE Control | LDPE (98%) | | | 2.00 | | | Without halo |
| PL21E10 | LDPE (68%) | 25.00 | | 2.00 | 5.00% | | With halo |
| PL21E11 | LDPE (70%) | 25.00 | | 2.00 | | 3.00% | With halo |

## Claims

1. **MESH MATERIALS FOR CONTROLLED RELEASE OF ANTIVIRALS,** wherein they consist of one or more mesh materials selected from the group consisting of polymers, fibers, non-woven fabric (NWF), fabrics, paper or cellulose or a combination of one or more mesh materials with adhesives, impregnated, extruded, saturated, coated or laminated with bis-biguanide:cyclodextrin inclusion compounds in the range of molar ratios between 1:1 and 1:4.

2. **THE MESH MATERIALS FOR CONTROLLED RELEASE OF ANTIVIRALS according to claim 1,** wherein bis-biguanide is selected from the group consisting of chlorhexidine, 1,6-bis-(2-ethyl-hexylbiguanide-hexane) dihydrochloride, 1,6-di-(*N*1,*N*1'-phenyldiguanide-*N*5,*N*5')-hexane tetrahydrochloride, 1,6-di-(*N*1,*N*1'-phenyl-*N*1,*N*1'-methyldiguanide-*N*5,*N*5')-hexane dihydrochloride, 1,6-di-(*N*1,*N*1'-o-chlorophenyldiguanide-*N*5,*N*5')-hexane dihydrochloride, 1,6-di-(*N*1,*N*1'-2,6-dichlorophenyldiguanide-*N*5,*N*5')-hexane dihydrochloride, 1,6-di-[*N*1,*N*1'-beta-(*p*-methoxyphenyl)-diguanide-*N*5,*N*5']-hexane dihydrochloride, 1,6-di-(*N*1,*N*1'-alpha-methyl-beta-phenyldiguanide-*N*5,*N*5')-hexane dihydrochloride, 1,6-di-(*N*1,*N*1'-*p-*nitrophenyldiguanide-*N*5,*N*5')-hexane dihydrochloride, Omega.'-di-(*N*1,*N*1'-phenyldiguanide-*N*5,*N*5')-*N*-propyl ether dihydrochloride, omega-di-(*N*1,*N*1'-*p-*chlorophenyldiguanide-*N*5,*N*5')-di-n-propyl ether tetrahydrochloride, 1,6-di-(*N*1,*N*1'-2,4-dichlorophenyldiguanide-*N*5,*N*5')-hexane tetrahydrochloride, 1,6-di-(*N*1,*N*1'-p-methylphenyldiguanide-*N*5,*N*5')-hexane dihydrochloride, 1,6-di-(*N*1,*N*1'-2,4,5-trichlorophenyldiguanide-*N*5,*N*5')-hexane tetrahydrochloride, 1,6-di-[*N*1,*N*1'-Alpha-(*p*-chlorophenyl)ethyldiguanide-*N*5,*N*5']-hexane dihydrochloride, omega,omega-di-(*N*1,*N*1'-*p*-chlorophenyldiguanide-*N*5,*N*5')-m-xylene, 1,12-di-(*N*1,*N*1'-*p*-chlorophenyldiguanide-*N*5,*N*5')-dodecane dihydrochloride, 1,10-di-(*N*1,*N*1'-phenyldiguanide-*N*5,*N*5')-decane tetrahydrochloride, 1,12-di-(*N*1,*N*1'-phenyldiguanide-*N*5,*N*5')-dodecane tetrahydrochloride, 1,6-di-(*N*1,*N*1'-*o*-chlorophenyldiguanide-*N*5,*N*5')-hexane dihydrochloride, 1,6-di-(*N*1,*N*1'-*p*-chlorophenyldiguanide-*N*5,*N*5')-hexane tetrahydrochloride, ethylene bis(1-tolyl-biguanide), ethylene bis(*p*-tolyl-biguanide), ethylene bis(3,5-dimethylphenyl biguanide), ethylene bis(p-tert-amylphenyl biguanide), ethylene bis(nonylphenyl biguanide), ethylene bis(phenylbiguanide), ethylene bis(*N*-butylphenyl biguanide), ethylene bis(2,5-diethoxyphenyl biguanide), ethylene bis(2,4-dimethylphenyl biguanide), ethylene bis(*o*-diphenylbiguanide), ethylene bis(mixed amyl-naphthyl biguanide), *N*-butyl ethylene bis(phenyl biguanide), trimethylene bis(o-tolyl biguanide), *N*-butyl trimethylene bis(phenyl biguanide), and the corresponding pharmaceutically acceptable salts of all the items above, such as acetates, gluconates, hydrochlorides, hydrobromides, citrates, bisulfites, fluorides, polyhalates, *N-*alkylalkylsarcosinates, phosphites, hypophosphites, perfluorooctanoates, silicates, sorbates, salicylates, maleates, tartrates, fumarates, ethylenediaminetetraacetates, iminodiacetates, cinnamates, thiocyanates, arginates, pyrromellites, tetracarboxybutyrates, benzoates, glutarates, monofluorophosphates, and perfluoropropionates and mixtures thereof.

3. **THE MESH MATERIALS FOR CONTROLLED RELEASE OF ANTIVIRALS according to claim 1,** wherein the cyclodextrin is selected from the group consisting of alpha-cyclodextrin, beta-cyclodextrin, gamma-cyclodextrin, hydroxy-propyl-beta-cyclodextrin, 2,6-o-methyl-beta-cyclodextrin, sulfobutyl-ether-beta-cyclodextrin, radomyl-beta-cyclodextrin, methyl-beta-cyclodextrin and maltosyl-beta-cyclodextrin.

4. **THE MESH MATERIALS FOR CONTROLLED RELEASE OF ANTIVIRALS according to claim 1,** wherein the inclusion compounds comprise chlorhexidine:cyclodextrin, in the molar ratio range between 1:1 and 1:4.

5. **THE MESH MATERIALS FOR CONTROLLED RELEASE OF ANTIVIRALS according to claim 4,** wherein the mesh materials are impregnated with at least 0.02% by mass of chlorhexidine.

6. **A PROCESS FOR OBTAINING MESH MATERIALS FOR THE CONTROLLED RELEASE OF ANTIVIRAL AGENTS defined in claim 1,** wherein it comprises the following steps:
a. Preparing an aqueous solution of inclusion compounds of bis-biguanide:cyclodextrin, in the molar ratio range between 1:1 and 1:4 (bis-biguanide:cyclodextrin), at room temperature, or up to 80 °C;
b. Impregnating, extruding, saturating, coating or laminating selected mesh materials from the group consisting of polymers, fibers, non-woven fabric (NWF), fabrics, paper or cellulose, alone or in combination, or a combination of these mesh materials with adhesives, with the aqueous solutions of step "a", in final mass concentrations of at least 0.02% of bis-biguanide, for a time of at least 4 seconds, using the methods of exhaustion, impregnation, saturation or spray, dyeing, serigraphy, printing, laundry or atomization, whether or not followed by squeezing and/or fixing;
c. Drying the mesh material obtained in step "b" at room temperature or at a temperature between 70 °C and 200 °C.

7. **THE PROCESS FOR OBTAINING MESH MATERIALS FOR CONTROLLED RELEASE OF ANTIVIRALS according to claim 6,** wherein, in step "a", the aqueous solution comprises varnishes, dyes or other actives compatible with the inclusion compounds.

8. **THE PROCESS FOR OBTAINING MESH MATERIALS FOR CONTROLLED RELEASE OF ANTIVIRALS according to claim 6,** wherein, in step "a", the cyclodextrin is selected from the group consisting of alpha-cyclodextrin, beta-cyclodextrin, gamma-cyclodextrin, hydroxy-propyl-beta-cyclodextrin, 2,6-o-methyl-beta-cyclodextrin, sulfobutyl-ether-beta-cyclodextrin, radomyl-beta-cyclodextrin, methyl-beta-cyclodextrin and maltosyl-beta-cyclodextrin, and bis- biguanide is selected from the group consisting of chlorhexidine, 1,6-bis-(2-ethyl-hexylbiguanide-hexane) dihydrochloride, 1,6-di-(*N*1,*N*1'-phenyldiguanide-*N*5,*N*5')-hexane tetra-hydrochloride, 1,6-di-(*N*1,*N*1'-phenyl-*N*1,*N*1'-methyldiguanide-*N*5,*N*5')-hexane dihydrochloride, 1,6-di-(*N*1,*N*1'-*o*-chlorophenyldiguanide-*N*5,*N*5')-hexane dihydrochloride, 1,6-di-(*N*1,*N*1'-2,6-dichlorophenyldiguanide-*N*5,*N*5')-hexane dihydrochloride, 1,6-di-[*N*1,*N*1'-beta-(p-methoxyphenyl)diguanide-*N*5,*N*5']-hexane dihydrochloride, 1,6-di-(*N*1,*N*1'-alpha-methyl-beta-phenyldiguanide-*N*5,*N*5')-hexane dihydrochloride, 1,6-di-(*N*1,*N*1'-*p*-nitrophenyldiguanide-*N*5,*N*5')-hexane dihydrochloride, omega-di-(*N*1,*N*1'-phenyldiguanide-*N*5,*N*5')-n-propyl ether dihydrochloride, omega'-di-(*N*1,*N*1'-p-chlorophenyldiguanide-*N*5,*N*5')-di-n-propyl ether tetrahydrochloride, 1,6-di-(*N*1,*N*1'-2,4-dichlorophenyldiguanide-*N*5,*N*5')-hexane tetrahydrochloride, 1,6-di-(*N*1,*N*1'-*p*-methylphenyldiguanide-*N*5,*N*5')hexane dihydrochloride, 1,6-di-(*N*1,*N*1'-2,4,5-trichlorophenyldiguanide-*N*5,*N*5')-hexane tetrahydrochloride, 1,6-di-[*N*1,*N*1'-alpha-(*p*-chlorophenyl)ethyldiguanide-*N*5,*N*5']-hexane dihydrochloride, omega-mega-di-(*N*1,*N*1'-*p*-chlorophenyldiguanide-*N*5,*N*5')-m-xylene, 1,12-di-(*N*1,*N*1')-*p*-chlorophenyldiguanide-*N*5,*N*5')-dodecane dihydrochloride, 1,10-di-(*N*1,*N*1'-phenyldiguanide-*N*5,*N*5')-decane tetrahydrochloride, 1,12-di-(*N*1,*N*1'-phenyldiguanide-*N*5,*N*5')-dodecane tetrahydrochloride, 1,6-di-(*N*1,*N*1'-*o*-chlorophenyldiguanide-*N*5,*N*5')-hexane dihydrochloride, 1,6-di(*N*1,*N*1'-p-chlorophenyldiguanide-*N*5,*N*5')-hexane tetrahydrochloride, ethylene bis(1-tolyl biguanide), ethylene bis(*p*-tolyl biguanide), ethylene bis(3,5-dimethylphenyl biguanide), ethylene bis(*p*-tert-amylphenyl biguanide), ethylene bis(nonylphenyl biguanide), ethylene bis(phenylbiguanide), ethylene bis(*N*-butylphenyl biguanide), ethylene bis(2,5-diethoxyphenyl biguanide), ethylene bis(2,4-dimethylphenyl biguanide), ethylene bis(*o*-diphenyl biguanide), ethylene bis(mixed amyl naphthyl biguanide), *N*-butyl ethylene bis (phenylbiguanide), trimethylene bis(o-tolyl biguanide), *N*-butyl trimethylene bis(phenyl biguanide), and the corresponding pharmaceutically acceptable salts of all of the items above, such as the acetates, gluconates, hydrochlorides, hydrobromides, citrates, bisulfites, fluorides, polyhalates, *N-*alkylsarcosinates, phosphites, hypophosphites, perfluorooctanoates, silicates, sorbates, salicylates, maleates, tartrates, fumarates, ethylenediaminetetraacetates, iminodiacetates, cinnamates, thiocyanates, arginates, pyrromellites, tetracarboxybutyrates, benzoates, glutarates, monofluorophosphates, and perfluoropropionates and mixtures thereof.

9. **A USE OF MESH MATERIALS FOR CONTROLLED RELEASE OF ANTIVIRALS defined in claim** 1, wherein it is as systems for the controlled release of antimicrobial and antiviral compounds, including against coronaviruses.

10. **THE USE OF MESH MATERIALS FOR CONTROLLED RELEASE OF ANTIVIRALS according to claim 9,** wherein it is as systems for the controlled release of antimicrobial and antiviral compounds, in air conditioning filters, disposable materials, personal protective equipment, packaging, clothes.
